# EUROPEAN PATENT APPLICATION

(11) **EP 4 653 459 A1**
(43) Date of publication of application: **26.11.2025**
(21) Application number: 23917910.4
(22) Date of filing: 20.12.2023
(51) Int. Cl.: C07K 16/28, A61P 29/00, A61K 39/00

(54) **ANTIBODY SPECIFICALLY BINDING TO IL-10 RECEPTOR AND USE THEREOF**

(30) Priority: 17.01.2023 KR 20230006450
(71) Applicant: IUCF-HYU (Industry-University Cooperation Foundation Hanyang University), Seoul 04763 (KR)
(72) Inventor: LEE, Keun Hwa, Incheon 21347 (KR)
(74) Representative: dompatent
(86) International application number: PCT/KR2023/021045
(87) International publication number: WO 2024/154958

(57) **Abstract**

The present invention relates to an antibody specifically binding to an IL-10 receptor and use thereof. The antibody targets IL-10 signaling and has an excellent effect of suppressing cytokine storm and excessive inflammation in severe inflammatory infectious diseases, and in particular, can be effectively used in suppressing inflammatory responses in severe SARS-CoV-2 infection and fatal SFTSV infection.

## Description

### [Technical Field]

This application claims priority to and the benefit of Korean Patent Application No. 10-2023-0006450, filed with the Korean Intellectual Property Office (KIPO) on January 17, 2023, the disclosure of which is incorporated herein by reference in its entirety.

The present invention relates to an antibody specifically binding to an IL-10 receptor protein and a use thereof.

### [Background Art]

A cytokine storm is a life-threatening systemic inflammatory syndrome that may cause life-threatening imbalanced immune responses, hyperactivation of immune cells, serious pathological changes, and multi-organ dysfunction, which result from excessive production of pro-inflammatory cytokines (*e.g*., interleukin-6 (IL-6)). In this case, the cytokine storm may cause serious pathological changes and result in multi-organ dysfunction.

Severe acute respiratory syndrome coronavirus 2 (SARS-CoV-2), which is a serious inflammatory infectious disease, severe fever with thrombocytopenia syndrome virus (SFTSV, recently named Dabie bandavirus), which is a newly emerging lethal tick-borne viral infection in Asia, and highly pathogenic avian influenza A (H5N1) virus may produce pro-inflammatory cytokines and cause a serious and sometimes fatal disease characterized by hyperinflammation having cytokine storm features.

Among the pro-inflammatory cytokines, IL-10 and IL-6 are regulatory cytokines that play pleiotropic roles in the immune system, and are known as important immunoregulatory cytokines. In particular, when infected with severe acute respiratory syndrome coronavirus 2 (SARS-CoV-2), severe fever with thrombocytopenia syndrome (SFTS), and highly pathogenic avian influenza virus (H5N1), which are fatal and serious inflammatory infectious diseases, the IL-10 level significantly increased compared to that in common mild and severe inflammatory diseases and non-fatal diseases, and IL-6 and IL-10 were overproduced in the body, thereby causing a cytokine storm, worsening pathological symptoms, and resulting in death.

Meanwhile, transforming growth factor-β (TGF-β) is a regulatory cytokine that has a key function in controlling inflammation. TGF-β is known as an immunosuppressive cytokine. TGF-β is known to mainly act on T cells to exert an immunosuppressive function.

Therefore, IL-10, IL-6, and TGF-β play important roles in the host immune response in severe inflammatory infectious diseases, and in particular, there is an increasing interest in therapeutic compositions for severe inflammatory responses and therapeutic methods using the same by regulating their responses.

### [Disclosure]

### [Technical Problem]

The present inventors have confirmed that an antibody or an antigen-binding fragment thereof that specifically binds to an IL-10 receptor protein has an excellent effect in inhibiting excessive inflammation such as a cytokine storm. Therefore, the present invention has been completed based on these facts.

The present invention is directed to providing a pharmaceutical composition for preventing or treating an inflammatory disease, which includes, as an active ingredient, an antibody or an antigen binding fragment thereof that specifically binds to an interleukin-10 (IL-10) receptor protein.

Also, the present invention is directed to providing a method of preventing or treating an inflammatory disease, which includes: administering an antibody or an antigen binding fragment thereof, which specifically binds to an interleukin-10 (IL-10) receptor protein, to a subject.

Further, the present invention is directed to providing a novel antibody or an antigen binding fragment thereof that specifically binds to an interleukin-10 (IL-10) receptor protein.

### [Technical Solution]

Hereinafter, the present invention will be described in more detail.

The present invention has confirmed that the levels of IL-6 and IL-10 are significantly higher and strongly produced in patients with severe and critical SARS-CoV-2 infection and fatal severe fever with thrombocytopenia syndrome. In contrast, the level of TGF-β was significantly lower in patients with severe acute respiratory syndrome coronavirus 2 and fatal severe fever with thrombocytopenia syndrome than in patients with mild/moderate SARS-CoV-2 infection and non-fatal severe fever with thrombocytopenia syndrome. Accordingly, the present inventors have confirmed that the production of IL-6 decreased and the production of TGF-β increased when the IL-10 signal was inhibited using an antibody or an antigen-binding fragment thereof that specifically binds to the interleukin-10 (IL-10) receptor protein.

Accordingly, the present invention provides a use of an antibody or an antigen-binding fragment thereof, which specifically binds to an interleukin-10 (IL-10) receptor protein, for the manufacture of a pharmaceutical composition for preventing or treating an inflammatory disease, a pharmaceutical composition for preventing or treating an inflammatory disease, which includes the antibody or antigen-binding fragment thereof that specifically binds to an interleukin-10 (IL-10) receptor protein as an active ingredient, and a method of preventing or treating an inflammatory disease, which includes: administering to a subject in need thereof an effective amount of the antibody or antigen-binding fragment thereof that specifically binds to an interleukin-10 (IL-10) receptor protein.

In the present specification, the term "antibody" is used interchangeably with the term "immunoglobulin (Ig)." Since the present invention relates to an antibody of the IL-10 receptor, the term "antibody" used without modification, unless otherwise specifically indicated, may refer to an IL-10 receptor antibody that specifically binds to an epitope of the IL-10 receptor. A complete antibody has a structure having two full-length light chains and two full-length heavy chains. Here, each of the light chains is linked to a heavy chain via a disulfide bond (SS-bond). The antibody may be an animal-derived antibody, a mousehuman chimeric antibody, a humanized antibody, or a human antibody.

In the present specification, the term "antigen-binding fragment" refers to a fragment of the entire antibody structure, that is, a portion of an antibody that contains a portion capable of binding to an antigen. For example, the antigen-binding fragment may be scFv, (scFv)2, Fv, Fab, Fab', Fv, F(ab')2, a diabody, a triabody, a tetrabody, Bis-scFv, a nanobody, or a combination thereof.

In the present specification, the "antibody" may refer to either an antibody or an antigen-binding fragment.

In the present specification, the term "antibody or antigen binding fragment thereof" may include not only the sequence of an IL-10 receptor antibody but also a biological equivalent thereof, and may be modified within the scope of specifically recognizing the IL-10 receptor. For example, the amino acid sequence of the antibody may be further changed to further improve the binding affinity and/or other biological properties of the antibody. Also, the antibody or antigen-binding fragment thereof may be modified by conjugation or linkage, glycosylation, tag attachment, or a combination thereof.

In the present invention, the term "antibody" refers to a specific protein molecule directed against an antigenic site. For the purposes of the present invention, the term "antibody" refers to an antibody that specifically binds to an IL-10 receptor, and includes polyclonal antibodies, monoclonal antibodies, and recombinant antibodies.

In the present specification, the term "epitope" refers to an antigenic determinant to which an antibody may specifically bind. The epitope is typically composed of chemically active surface molecules, such as amino acids or sugar side chains, and typically has specific three-dimensional structural characteristics as well as specific charge characteristics. Stereological and non-stereoscopic epitopes are distinguished in that binding to the former is lost in the presence of a denaturing solvent, but not to the latter.

The antibody or antigen-binding fragment thereof that specifically binds to the interleukin-10 (IL-10) receptor protein that may be used in the present invention is not particularly limited. An antibody or antigen-binding fragment thereof for a known IL-10 receptor protein may be used, or an antibody or antigen-binding fragment thereof newly prepared using the IL-10 receptor protein may be used.

In one specific embodiment of the present invention, the antibody or antigen-binding fragment thereof may specifically bind to the amino acid sequence of SEQ ID NO: 2 in the IL-10 receptor protein, but the present invention is not limited thereto. For example, the antibody or antigen-binding fragment thereof may consist of one or more amino acid sequences selected from the group consisting of a portion of the amino acid sequence (235 amino acids) of SEQ ID NO: 2, for example, one or more amino acid sequences selected from the group consisting of a peptide consisting of amino acids 1 to 235, a peptide consisting of amino acids 1 to 230, a peptide consisting of amino acids 5 to 235, a peptide consisting of amino acids 5 to 230, a peptide consisting of amino acids 10 to 235, a peptide consisting of amino acids 10 to 230, a peptide consisting of amino acids 15 to 235, a peptide consisting of amino acids 15 to 230, a peptide consisting of amino acids 20 to 235, a peptide consisting of amino acids 20 to 230, and a peptide consisting of amino acids 22 to 235, but the present invention is not limited thereto.

In another specific embodiment, the antibody or antigen binding fragment thereof may specifically bind to the amino acid sequence of SEQ ID NO: 3 in the IL-10 receptor protein.

In still another specific embodiment, the antibody or antigen binding fragment thereof may specifically bind to the amino acid sequence of SEQ ID NO: 4 in the IL-10 receptor protein.

As described above, the antibody or antigen-binding fragment thereof that specifically binds to the interleukin-10 (IL-10) receptor protein may be used as an active ingredient for preventing or treating an inflammatory disease.

The term "prevention" refers to any action that inhibits or delays the progression of an inflammatory disease, particularly a disease associated with the activation or overproduction of IL-10 receptors (*e.g*., severe acute respiratory syndrome coronavirus 2 (SARS-CoV-2) infection, severe fever with thrombocytopenia syndrome (SFTS)) by administering the pharmaceutical composition, and the term "treatment" refers to any action that inhibits, alleviates or eliminates a disease associated with the activation or overproduction of IL-10 receptors (e.g., severe acute respiratory syndrome coronavirus 2, severe fever with thrombocytopenia syndrome).

In the present invention, the inflammatory disease may be a disease caused by one or more selected from the group consisting of severe acute respiratory syndrome coronavirus 2 (SARS-CoV-2), severe fever with thrombocytopenia syndrome (SFTS) virus, and highly pathogenic avian influenza A (H5N1), but the present invention is not limited thereto.

In the present specification, the term "severe acute respiratory syndrome coronavirus 2" refers to severe acute respiratory syndrome coronavirus 2 (SARS-CoV-2), that is, an enveloped virus having a positive-strand RNA genome of approximately 30,000 base pairs. Since it was first reported in Wuhan, China in December 2019, the number of infected patients has been increased explosively worldwide. Analysis of the viral genetic sequence showed that it is a virus having a similarity of approximately 79% to the severe acute respiratory syndrome virus (SARS, SARS virus) and a similarity of approximately 50% to the Middle East respiratory syndrome virus (MERS, MERS virus). Coronaviruses express spike proteins on their viral surface, which bind to receptors and induce infection in host cells.

The term "severe fever with thrombocytopenia syndrome (SFTS)" used in the present specification was first made known to the world in 2011 when a report was made in the New England Journal of Medicine (NEJM) of a case in which patients having high fever and thrombocytopenia of unknown etiology as the main symptoms appeared in six provinces (Liaoning, Shandong, Henan, Hubei, Anhui, and Jiangsu) in central-eastern and northern China in 2009 and 2010. The SFTS virus, transmitted by the small cattle tick (*Haemaphysalis longicornis*), causes high fever and, in severe cases, is characterized by fever, respiratory or gastrointestinal symptoms, thrombocytopenia, leukopenia, and hemorrhages and has a mortality of approximately 6 to 30%.

In the present specification, the term "avian influenza" refers to a disease caused by an influenza virus. Here, the influenza virus is an RNA virus belonging to the *Orthomyxoviridae* family and consists of subtypes named A, B, and C. Among them, influenza A is known to cause infection in humans, horses, pigs, other mammals, and various types of poultry and wild birds, and is divided into 16 types from H1 to H16 depending on the characteristics of hemagglutinin present on the surface of the virus and 9 types from N1 to N9 depending on the characteristics of the surface protein expressed by the enzyme called neuraminidase. When these H and N types are combined, theoretically, there are a total of 144 subtypes of influenza A viruses. Among these influenza A types, avian influenza H9N2 and H5N1 viruses that cause infection in birds are continuously causing infection in poultry in Korea. Highly pathogenic avian influenza H5N1 has been reported to infect humans and cause fatal outcomes. After the first human infection was reported in Hong Kong in 1997, reports of human infections began to increase rapidly after 2003 when the frequency of outbreaks in poultry increased rapidly.

Also, the present invention provides a novel antibody or antigen-binding fragment thereof that specifically binds to an interleukin-10 (IL-10) receptor protein.

In the present invention, the definition of the antibody and antigen binding fragment is as described above.

The novel antibody or antigen-binding fragment thereof according to the present invention may specifically bind to the amino acid sequence of SEQ ID NO: 2 in the IL-10 receptor protein.

In another specific embodiment, the antibody or antigen-binding fragment thereof may specifically bind to the amino acid sequence of SEQ ID NO: 3 in the IL-10 receptor protein.

In still another specific embodiment, the antibody or antigen-binding fragment thereof may specifically bind to the amino acid sequence of SEQ ID NO: 4 in the IL-10 receptor protein.

The antibody or antigen-binding fragment thereof that specifically binds to the interleukin-10 (IL-10) receptor protein is formulated into a pharmaceutical composition together with a pharmaceutically acceptable carrier, and administered to a subject in need thereof.

The pharmaceutically acceptable carrier refers to a diluent, adjuvant, excipient, or vehicle with which the antibody is administered. Some examples of the carrier are water, saline, phosphate buffered saline, dextrose, glycerol, ethanol, and combinations thereof. In many cases, an isotonic agent, for example, a sugar, a polyalcohol (*e.g*., mannitol, sorbitol), or sodium chloride, is preferably included in the composition.

The pharmaceutical composition may contain pharmaceutically acceptable auxiliary substances required to approximate physiological conditions, such as pH adjusters and buffers, stabilizers, thickeners, lubricants, and colorants.

In the present specification, the terms "administration" and "treatment" refer to any action that internally or externally administers a composition, such as a composition of the present invention, to a patient having one or more disease symptoms, wherein the therapeutic agent has known therapeutic activity. Typically, the agent is administered in an amount effective to alleviate one or more disease symptoms in the patient or population being treated, either by inducing regression of the symptom(s) or by inhibiting their progression to any clinically measurable degree. The amount of therapeutic agent effective for alleviating any particular disease symptom (also referred to as a "therapeutically effective amount") may vary depending on factors such as the disease state, age, and weight of a patient, and the ability of the drug to elicit a desired response in the patient. It refers to the contact of an exogenous agent, treatment, diagnostic agent, or composition with an animal, human, test subject, cell, tissue, organ, or biological fluid.

In the present specification, the term "effective amount" as used herein refers to an amount sufficient to exhibit a preventive or therapeutic effect when administered to a subject in need of prevention or treatment. The effective amount varies depending on factors such as a formulation method, the mode of administration, the age, weight, sex, and pathological condition of a patient, food, an administration time, a route of administration, an excretion rate, and response sensitivity, and may be appropriately selected by a person skilled in the art depending on the selected cell or subject. For example, the daily dose of the pharmaceutical composition may be in the range of 3 to 10 mg/kg, 3 to 8 mg/kg, 3 to 6 mg/kg, 5 to 10 mg/kg, 5 to 8 mg/kg, 5 to 6 mg/kg, for example, 6 mg/kg. The pharmaceutical composition may be administered once a day, several times a day, or once a week, once every two weeks, once every three weeks, or once every four weeks to once a year.

Another aspect of the present invention is a method of preventing or treating an inflammatory disease, particularly a disease associated with the activation or overproduction of IL-10 receptors (e.g., severe acute respiratory syndrome coronavirus 2 (SARS-CoV-2) infection, severe fever with thrombocytopenia syndrome (SFTS)), which includes administering an effective amount of an antibody or an antigen-binding fragment thereof that specifically binds to an interleukin-10 (IL-10) receptor protein to a subject.

In the present invention, the subject may be a mammal, for example, a human, a cow, a horse, a pig, a dog, a sheep, a goat, or a cat. The subject may be a subject that is highly likely to develop a disease associated with the activation or overproduction of IL-10, for example, severe acute respiratory syndrome coronavirus 2 (SARS-CoV-2) infection, severe fever with thrombocytopenia syndrome (SFTS).

In the present specification, any contents overlapping with the composition in the method of preventing or treating an inflammatory disease is omitted.

### [Advantageous Effects]

The present invention relates to an antibody that specifically binds to an IL-10 receptor and a use thereof. The antibody effectively inhibits IL-10 signaling, and thus has an excellent effect of suppressing a cytokine storm and excessive inflammation in serious inflammatory infectious diseases.

### [Description of Drawings]

FIG. 1 is a graph showing the results of measuring the levels of IL-6, IL-10, and TGF-β in the serum of patients with mild/moderate and severe/critical severe acute respiratory syndrome coronavirus 2 (SARS-CoV-2) infection according to one embodiment of the present invention.
FIG. 2 is a graph showing the results of measuring the levels of IL-2, IL-4, and IL-17A in the serum of patients with mild/moderate and severe/critical SARS-CoV-2 infection according to one embodiment of the present invention.
FIG. 3 is a graph showing the results of measuring the levels of IFN-γ and TNF-α in the serum of patients with moderate SARS-CoV-2 infection and patients with severe/critical SARS-CoV-2 infection according to one embodiment of the present invention.
FIG. 4 is a graph showing the results of measuring the levels of IL-6, IL-10, and TGF-β in the serum of non-fatal and fatal SFTS patients according to one embodiment of the present invention.
FIG. 5 is a graph showing the results of measuring the levels of IL-2, IL-4, and IL-17A in the serum of non-fatal and fatal SFTS patients according to one embodiment of the present invention.
FIG. 6 is a graph showing the results of measuring the levels of IFN-γ and TNF-α in the serum of non-fatal and fatal SFTS patients according to one embodiment of the present invention.
FIG. 7 is a graph showing the results of measuring the expression levels of IL-6, IL-10, and TGF-β in LPS-induced THP-1-derived macrophages treated with IL-6R and IL-10RA polyclonal antibodies according to one embodiment of the present invention.
FIG. 8 is a graph showing the results of measuring the expression levels of IL-6, IL-10, and TGF-β when THP-1-derived macrophages infected with SARS-CoV-2 according to one embodiment of the present invention were treated with an IL-10RA polyclonal antibody.
FIG. 9 is a graph showing the results of measuring the expression levels of IL-6, IL-10, and TGF-β when THP-1-derived macrophages infected with SFTS virus according to one embodiment of the present invention were treated with an IL-10RA polyclonal antibody.
FIG. 10 is a graph showing the results of measuring the expression levels of IL-6 and IL-10 in the LPS-induced THP-1-derived macrophages treated with IL-6R and IL-10RA polyclonal antibodies by varying the types of IL-10RA polyclonal antibodies according to one embodiment of the present invention.

### [Mode for Invention]

Hereinafter, one or more specific embodiments will be described in more detail through examples. However, it should be understood that these examples are merely provided to illustrate one or more specific embodiments, and are not intended to limit the scope of the present invention.

### Example 1: Setup of experimental group

### 1-1. Establishment of COVID-19 patient data

A retrospective study of COVID-19 patients was conducted from August 2020 to July 2021. Data from 109 of 188 patients confirmed to have SARS-CoV-2 infection was extracted.

Data on demographics and clinical characteristics included patient demographics (age, sex, body mass index (BMI)), past medical history and Charlson comorbidity index (CCI), risk factors for exposure to SARS-CoV-2, onset of symptoms, oxygen therapy, hospital treatment (inotropic agents, extracorporeal membrane oxygenation), hospitalization period, and mortality. Laboratory variables were obtained retrospectively, and disease severity was classified into five groups according to the National Institutes of Health criteria based on the patient's worst condition during hospitalization:
Asymptomatic, Mild, Moderate, Severe, and Critical.
[Reference: National Institutes of Health (NIH). Clinical spectrum of SARS-CoV-2 infection. Available at: https://www.covid19treatmentguidelines.nih.gov/overview/clinical-spectrum/. Accessed June 25, 2022].

According to the analysis results, patients confirmed to have SARS-CoV-2 infection had the clinical characteristics listed in Table 1.

**[Table 1]**

| Variables | Total (*n* = 109) | Mild (*n* = 40) | Moderate (*n* = 40) | Severe/Critical (*n* = 29) | *P* |
|---|---|---|---|---|---|
| Gender: Male (%) | 53 (48.6) | 15 (37.5) | 19 (47.5) | 19 (65.5) | 0.054 |
| Age (Years) | 51.1 ± 19.9 | 37.1 ± 23.1 | 57.0 ± 11.7* | 62.2 ± 11.9* | < 0.001 |
| BMI (kg/m²) | 24.7 ± 5.2 | 22.4 ± 6.7 | 25.5 ± 3.6† | 26.4 ± 3.9† | 0.03 |
| CCI | 1.8 + 1.8 | 0.9 + 1.4 | 2.2 ± 1.9‡ | 2.6 ± 1.8‡ | < 0.001 |
| SARS-CoV-2 RT PCR (Ct) | 22.0 ± 6.8 | 22.6 ± 6.7 | 20.6 ± 5.8 | 23.2 ± 8.0 | 0.19 |
| Time from Symptoms to Diagnosis (Years) | 1.5 ± 3.3 | 1.5 ± 2.4 | 1.7 ± 2.7 | 1.1 ± 4.9 | 0.79 |
| BT (°C) | 36.5 ± 8.4 | 36.4 ± 7.5 | 37.1 ± 7.3 | 35.9 ± 10.4 | 0.86 |
| O₂ Supply Mode (%) | | | | | < 0.001 |
| No | 82 (75.2) | 40 (100) | 39 (100) | 3 (10.3) | |
| Low Flow | 21 (19.4) | 0 | 0 | 21 (72.4) | |
| High Flow | 4 (3.7) | 0 | 0 | 4 (13.8) | |
| MV | 1 (0.9) | 0 | 0 | 1 (3.4) | |
| ECMO (%) | 0 | 0 | 0 | 0 | |
| Vasopressor (%) | 2 (1.9) | 0 | 0 | 2 (6.9) | 0.13 |
| Treatment (%) | | | | | |
| HCQ | 1 (0.9) | 0 | 1 (2.5) | 0 | 0.50 |
| Lopinavir/Ritonavir | 21 (19.4) | 0 | 6 (15.0) | 15 (51.7) | < 0.001 |
| Regdanvimab | 42 (38.5) | 8 (20.0) | 28 (70.6) | 6 (20.7) | < 0.001 |
| Remdesivir | 18 (16.7) | 0 | 2 (5.0) | 16 (55.2) | < 0.001 |
| Steroid | 28 (25.9) | 0 | 6 (15.0) | 22 (75.9) | < 0.001 |
| Tocilizumab | 0 | 0 | 0 | 0 | N/A |
| WBC (x10³/µL) | 5395 ± 2939 | 4948 + 2369 | 5410 ± 2470 | 5857 ± 4146 | 0.40 |
| Hb (g/dL) | 7.3 ± 7.1 | 9.8 ± 6.7 | 7.4 ± 10.5 | 7.3 ± 1.9 | 0.30 |
| PLT (x10⁶/µL) | 193 + 70 | 206 + 79** | 207 ± 62** | 156 ± 52 | 0.003 |
| AST (IU/L) | 41.6 ± 43.6 | 32.1 ± 40.1†† | 37.1 ± 25.5†† | 60.3 ± 59.2 | 0.02 |
| ALT (IU/L) | 33.6 ± 29.3 | 25.6 ± 40.9 | 37.3 ± 31.8 | 39.6 ± 29.8 | 0.09 |
| BUN (mg/dL) | 14.5 ± 5.5 | 15.3 ± 5.4 | 4.5 ± 6.4 | N/A | 0.38 |
| Cr (mg/dL) | 0.8 ± 0.2 | 0.7 ± 0.2 | 0.8 ± 0.2‡‡ | 0.8 ± 0.2‡‡ | 0.01 |
| CRP (mg/dL) | 2.6 ± 4.9 | 0.4 ± 0.6*** | 2.3 ± 4.6*** | 5.7 ± 6.6 | < 0.001 |
| Mortality (%) | 1 (0.9) | 0 | 0 | 1 (3.4) | 0.48 |
| Duration of Administration | 12.5 ± 5.2 | 11.4 ± 2.4††† | 11.7 ± 3.8††† | 15.0 ± 8.1 | 0.009 |

| | | | | | |
|---|---|---|---|---|---|
| Data is presented as mean ± standard deviation. *, †, and ‡ indicate no significant differences between groups. SD = Standard Deviation, BMI = Body Mass Index, CCI = Charlson Comorbidity Index Score, SARS-CoV-2 = Severe Acute Respiratory Syndrome Coronavirus 2, Real-Time RTPCR = Real-Time Reverse Transcriptase-Polymerase Chain Reaction, Ct = Cycle Threshold, BT = Body Temperature, MV = Mechanical Ventilation, ECMO = Extracorporeal Membrane Oxygenation, HCQ = Hydroxychloroquine, WBC = White Blood Cell Count, Hb = Hemoglobin, PLT = Platelets, AST = Aspartate Aminotransferase, ALT = Alanine Aminotransferase, BUN = Blood Urea Nitrogen, Cr = Creatinine, CRP = C-Reactive Protein. | | | | | |

As shown in Table 1 above, among the 109 patients, 40 patients (32.5%) had a mild disease, 40 patients (17.5%) had a moderate disease, 27 patients (45%) had a severe disease, and 2 patients (5%) had a critical disease. The mean age of the patients was 51.1 ± 19.9 years, and 53 patients (48.6%) were male. No patients were vaccinated because the COVID-19 vaccine was not introduced in Korea during the study period. The mean body mass index (BMI) of the patients was 24.7 kg/m². The lowest value of the mean Charlson comorbidity index (CCI) was 1.8 ± 1.8. The time from the onset of symptoms to the SARS-CoV-2 RT-PCR test was 1 day, and the mean SARS-CoV-2 RT-PCR cycle threshold (Ct) value was 22.0 ± 6.8.

The types of oxygen supplementation received by patients in the study were as follows: 82 patients (75.2%) received no oxygen, 21 patients (19.4%) received low-flow nasal cannula therapy and a face mask with a reservoir, 4 patients (3.7%) received high-flow nasal cannula therapy, and 1 patient (0.9%) received invasive mechanical ventilation. Hydroxychloroquine, lopinavir/ritonavir, remdesivir, and corticosteroids were administered to 1 patient (0.9%), 21 patients (19.4%), 18 patients (16.7%), and 28 patients (25.9%), respectively. The overall mortality was 0.9% and the mean number of days of hospitalization was 12.5 ± 5.2 days. In the comparison of the mild/moderate and severe/critical groups, the patients were more likely to be in the severe/critical group rather than the mild/moderate group when the patients were older, had a higher BMI, and had a higher CCI. There was no significant difference in the mean SARS-CoV-2 RT-PCR Ct value between the mild/moderate and severe/critical groups. High-flow invasive mechanical ventilation, lopinavir/ritonavir, remdesivir, and steroids were administered to the severe/critical group rather than the mild/moderate group. There was no significant difference in the experimental results between the groups. It was shown that C-reactive protein (CRP) was higher in the severe/critical group than in the mild/moderate group.

### 1-2. Establishment of data on patients with severe fever with thrombocytopenia syndrome

As the severe fever with thrombocytopenia syndrome (SFTS) patients, a group of 65 patients were analyzed who were hospitalized between May 2013 and April 2022 with a confirmed SFTS diagnosis and tested positive for the small (S) and large (L) segments of SFTSV RNA by real-time RT-PCR according to the diagnostic criteria recommended in Korea.

Data on demographics and clinical characteristics was obtained from electronic medical records, and included patient demographics, activity at the time of exposure, tick bite history, presence of initial symptoms, vital signs, past medical history, CCI, multiple organ dysfunction (MODS) during hospitalization and 72 hours after treatment (inotropic agents, intubation, renal replacement therapy, and plasma exchange), course after hospitalization, and 30-day mortality. Blood samples were collected at the patient's first hospital visit, and then collected periodically (every 2 days during the acute phase, twice weekly during admission during the recovery phase, and every 2 to 3 months after discharge from the hospital).

Patients were divided into a severe group (SG) and a non-severe group (NSG). SG was defined as patients with 3 or more organ failures based on the sequential organ failure assessment (SOFA) score, including: patients with pulmonary PaO₂/FiO₂ < 400 or liver serum bilirubin ≥ 1.2 mg/dL, or requiring mechanical ventilation; patients with kidney serum creatinine ≥ 1.2 mg/dL, urine output < 500 mL/day, or requiring hemodialysis for cardiovascular diseases; patients with mean arterial pressure < 70 mmHg or requiring vasopressors; and patients with Glasgow Coma Scale < 15 in the central nervous system, or death (see https://doi.org/10.3947/ic.2022.0073.). Also, the non-severe group represents a group of patients other than the severe group. The characteristics of each classified group are shown in Table 2 below.

**[Table 2]**

| Variables | Total (*n* = 65) | Non-critical Group (*n* = 58) | Critical Group (*n* = 7) | *P* |
|---|---|---|---|---|
| Gender: Male (%) | 37 (56.9) | 31 (53.4) | 6 (85.7) | 0.10 |
| Age (Years) | 63.7 ± 14.0 | 62.7 ± 14.1 | 72.0 ± 9.9 | 0.09 |
| CCI | 0.5 ± 0.8 | 0.4 ± 0.7 | 0.9 ± 0.9 | 0.15 |
| Exposure Type, n (%) | | | | 0.06 |
| Agricultural Activity | 37 (57.0) | 33 (56.9) | 4 (57.2) | |
| Outdoor Activity | 19 (28.3) | 17 (29.3) | 19 (29.3) | |
| Contact with SFTS Patients | 2 (3.1) | 2 (3.4) | 0 | |
| Others | 7 (10.8) | 6 (10.3) | 1 (14.3) | |
| Tick Bite Experience, n (%) | 28 (43.1) | 25 (43.1) | 3 (42.9) | 0.94 |
| Seasonal Occurrence, n | | | | 0.60 |
| (%) | | | | |
| Spring | 9 (13.8) | 7 (12.1) | 2 (28.6) | |
| Summer | 37 (56.9) | 33 (56.9) | 4 (57.1) | |
| Fall | 18 (27.7) | 17 (29.3) | 2 (14.3) | |
| Winter | 1 (1.5) | 1 (1.7) | 0 | |
| Duration from Symptoms to Diagnosis (Days) | 5.8 ± 5.3 | 5.8 ± 5.6 | 5.9 ± 2.9 | 0.96 |
| BT (°C) | 38.5 ± 0.8 | 38.6 ± 0.8 | 38.1 ± 0.9 | 0.17 |
| Duration of Fever (Days) | 4.4 ± 3.1 | 4.2 ± 2.9 | 6.0 ± 4.9 | 0.18 |
| WBC (Cells/µL) | 2,376.0 + 1,725.8 | 2,230.7 ± 1,590.1 | 3,580.0 ± 2,419.8 | 0.05 |
| ANC (Cells/µL) | 1,472.2 + 1,401.6 | 1,456.2 ± 1,413.8 | 1,621.8 ± 1,395.5 | 0.78 |
| Hb (g/dL) | 13.9 + 2.5 | 13.8 ± 2.6 | 15.3 ± 1.4 | 0.13 |
| PLT (Cells/µL) | 83,861 ± 37767 | 84,328.6 ± 34,579.4 | 800,000.0 ± 61,935.5 | 0.77 |
| AST (IU/L) | 198.8 + 330.7 | 153.3 ± 240.6 | 575.6 ± 656.5 | 0.001 |
| ALT (IU/L) | 83.7 ± 104.1 | 74.2 ± 88.4 | 162.3 ± 182.8 | 0.03 |
| Cr (mg/dL) | 1.2 ± 0.5 | 1.1 ± 0.5 | 1.3 ± 0.5 | 0.36 |
| PT, INR | 1.4 ± 1.8 | 1.4 ± 1.9 | 1.3 ± 0.4 | 0.84 |
| aPTT, sec | 40.9 ± 11.7 | 39.1 ± 7.2 | 56.3 ± 25.1 | < 0.001 |
| LDH, IU/L | 1,045.3 ± 1232.3 | 831.3 ± 653.0 | 3,042.7 ± 2921.6 | < 0.001 |
| CK, IU/L | 987.0 ± 1634.0 | 872.9 ± 1480.4 | 1834.3 ± 2496.0 | 0.14 |
| CRP (mg/dL) | 1.2 ± 2.2 | 1.1 ± 2.2 | 1.8 ± 2.6 | 0.46 |
| SFTS Viral Load | 82,115,042.6 + 643,558,056.7 | 220,657.9 ± 519,022.8 | 175,570,922.6 ± 1,914,726,025 | 0.004 |
| MODS Score | 2.7 + 2.5 | 2.3 ± 1.7 | 6.6 ± 4.7 | < 0.001 |
| ICU Care | 30 (46.2) | 23 (39.7) | 7 (100) | 0.002 |
| Vasopressor | 10 (15.4) | 4 (6.9) | 6 (85.7) | < 0.001 |
| Mechanical Ventilation | 7 (10.8) | 1 (1.7) | 6 (85.7) | < 0.001 |
| CRRT | 5 (7.7) | 1 (1.7) | 4 (57.1) | < 0.001 |
| Immunoglobulin | 2 (3.1) | 1 (1.7) | 1 (14.3) | 0.06 |
| Plasmapheresis | 26 (40.0) | 22 (37.9) | 4 (57.1) | 0.33 |
| Tocilizumab | 1 (1.5) | 1 (1.7) | 0 | 0.73 |
| Admission Days | 8.8 + 5.2 | 0 | 0 | 0.48 |
| 30-Day Mortality (%) | 52 (86.7) | 0 | 7 (100) | |

| | | | | |
|---|---|---|---|---|
| Data is presented as mean ± standard deviation. P values were calculated from t-tests between the recovery and death groups. SD = Standard Deviation, BMI = Body Mass Index, CCI = Charlson Comorbidity Index Score, WBC = White Blood Cell Count, ANC = Absolute Neutrophil Count, Hb = Hemoglobin, CRP = C-Reactive Protein, PT = Prothrombin Time, INR = International Normalized Ratio, aPTT = Activated Partial Thromboplastin Time, AST = Aspartate Aminotransferase, ALT = Alanine Aminotransferase, LDH = Lactate Dehydrogenase, CK = Creatinine Kinase, BUN = Blood Urea Nitrogen, MODS = Multiple Organ Dysfunction Syndrome, CRRT = Continuous Renal Replacement Therapy. | | | | |

As shown in Table 2 above, among the patients, 37 patients (56.9%) were male and had a mean age of 63.7 ± 14.0 years. All patients resided on Jeju Island. The mean CCI was very low at 0.5 ± 0.8. Most of the patients (85%) were exposed to the virus through occupational or outdoor activities, with the agricultural activity being the most common exposure type. Some patients did not know whether they had been bitten by a tick. Most cases occurred in summer, followed by fall. The mean duration from symptoms to SFTS diagnosis was 5.8 ± 3.3 days, and most cases were confirmed within 2 days of admission. The mean MODS was 2.7 ± 2.5, the mean SFTS viral load was 82,115,042 ± 643,558,056.30, the daily mortality was 13.4%, and 30 (46.2%) patients were admitted to the ICU. The initial laboratory tests showed neutropaenia, thrombocytopaenia, elevated liver enzymes, elevated creatine kinase CK and lactate dehydrogenase LDH, and prolonged activated partial thromboplastin time (aPTT).

When comparing the SFTS recovery patients and the death group, the male and older age groups tended to be included in the death group. There was no significant difference in comorbidity, exposure type, tick bite history, or seasonal occurrence in each group. However, SFTS mortality tended to increase in spring. High viral load and MODS were more associated with the death group than with the recovery group. Vasopressors, mechanical ventilation, and CRRT were applied to the death group. There was no significant difference between the groups in the application of therapeutic plasma exchange, immunoglobulin, or anti-IL-6 antagonists. Most critical cases died within 1 week of admission. The initial levels of neutropenia and thrombocytopenia could not distinguish whether the SFTS patients were included in the recovery or death group. However, liver enzymes, aPTT, CK, and LDH were significantly different between the two groups.

### Example 2: Serum cytokine analysis

To characterize SARS-CoV-2 and SFTSV infection in terms of the production of serum cytokines in COVID-19 and SFTS patients, a human Th1/Th2/Th17 CBA kit (BD Biosciences, San Diego, CA) was used to measure IL-2, IL-4, IL-6, IL-10, IL-17A, interferon-γ (IFN-γ), and tumor necrosis factor (TNF-α) according to a slightly modified method from the manufacturer's instructions.

Clinical specimens were obtained using a FACS Canto II flow cytometer and analyzed by FCAP Array software version 3.0 (BD Biosciences). All statistical analyses were performed using SPSS version 20.0 (IBM Corp.). TGF-β was measured in the collected serum using a human TGF-β-1 ELISA kit (Thermo Fisher Scientific) according to the manufacturer's protocol.

To compare the mean differences between mild/moderate and severe/critical COVID-19 patients and critical and non-critical SFTS disease patients, a two-sample t-test was usually used. When using this method, several assumptions such as normality, equal variance, and independence were checked. When these assumptions were not satisfied, a nonparametric 2-sample t-test, called the Wilcoxon-Mann Whitney test, was used. A P value < 0.05 indicates statistical significance.

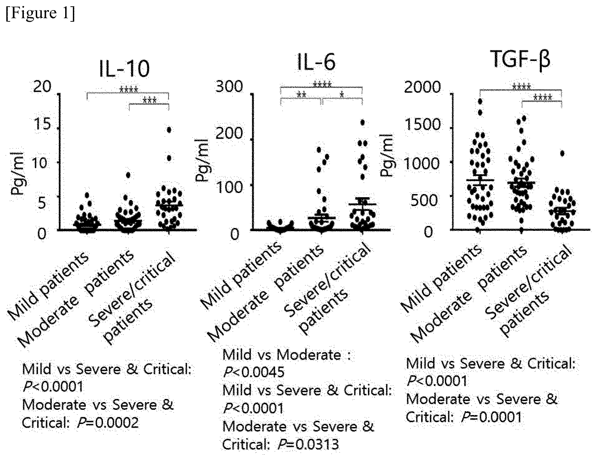

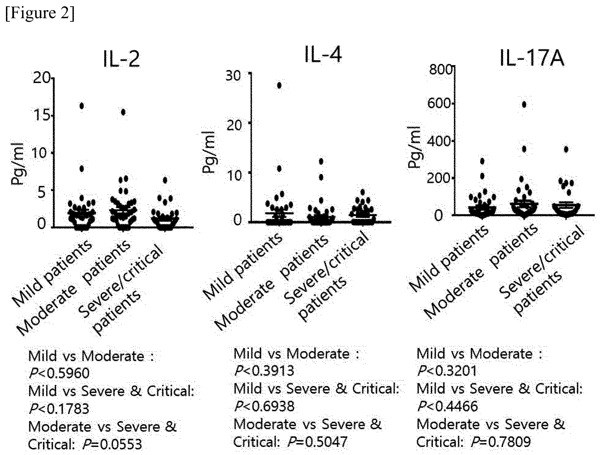

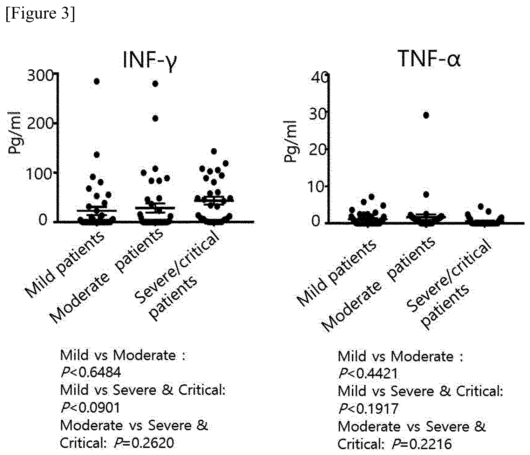

As shown in Table 3 below and FIGS. 1 to 3, serum IL-6 and IL-10 concentrations were significantly higher in the severe/critical COVID-19 patients compared to the mild/moderate COVID-19 patients, and TGF-β concentrations were significantly lower in the severe/critical disease patients compared to the mild/moderate disease patients during the early clinical course of hospitalization (FIG. 1). However, the differences in serum IL-2, IL-4, IL-17A, IFN-γ, and TNF-α levels between the severe/critical disease patients and the mild/moderate disease patients were not statistically significant (FIGS. 2 and 3).

**[Table 3]**

| Classification | Mild Patients | Moderate Patients | Severe/Critical Disease Patients | Mild vs Severe/Fatal p-Value | Mild vs Severe/Fatal p-Value |
|---|---|---|---|---|---|
| IL-2 | 2.0 (0.0 - 16.3) | 2.3 (0.0 - 15.5) | 1.2 (0.0 - 6.3) | 0.1783 | 0.0553 |
| IL-4 | 1.8 (0.0 - 27.6) | 1.1 (0.0 - 12.3) | 1.4 (0.0 - 6.1) | 0.6938 | 0.5047 |
| IL-6 | 5.3 (0.0 - 19.2) | 27.4 (0.0 - 177.8) | 58.2 (1.1 - 237.2) | < 0.0001 | 0.0313 |
| IL-10 | 0.8 (0.0 - 5.2) | 1.4 (0.0 - 8.1) | 3.7 (0.2 - 14.8) | < 0.0001 | 0.0002 |
| IL-17A | 42.2 (2.2 - 290.9) | 61.8 (0.0 - 595.2) | 55.1 (4.3 - 354.3) | 0.4466 | 0.7809 |
| IFN-γ | 22.9 (0.0 - 284.4) | 28.7 (0.0 - 289.7) | 43.6 (0.0 - 143.3) | 0.0901 | 0.2620 |
| TNF | 1.1 (0.0 - 7.1) | 1.7 (0.0 - 29.2) | 0.6 (0.0 - 4.6) | 0.1917 | 0.2216 |
| TGF-β | 731.5 (0.0 - 1893.3) | 697.6 (0.0 - 1640.0) | 282.5 (0.0 - 1126.7) | < 0.0001 | < 0.0001 |

| | | | | | |
|---|---|---|---|---|---|
| Units: pg/mL; Unless otherwise stated, values are listed as median and range. | | | | | |

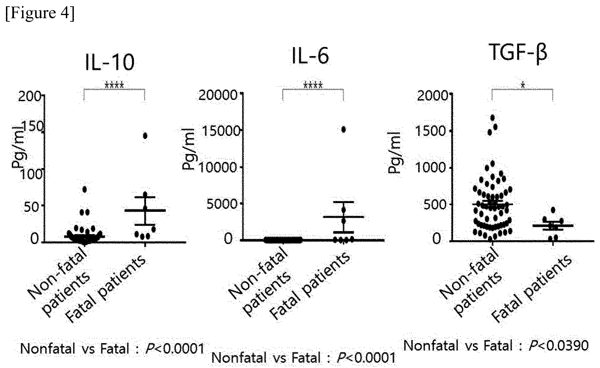

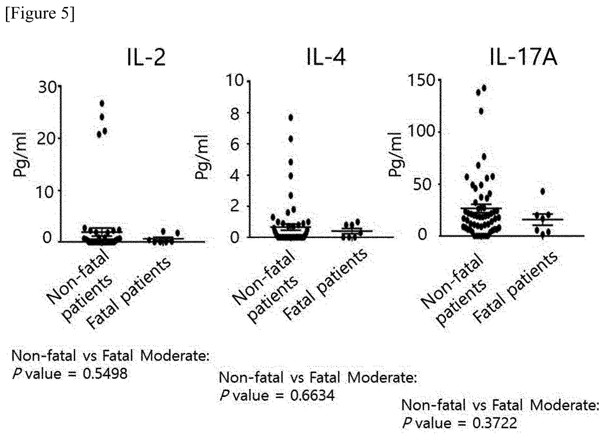

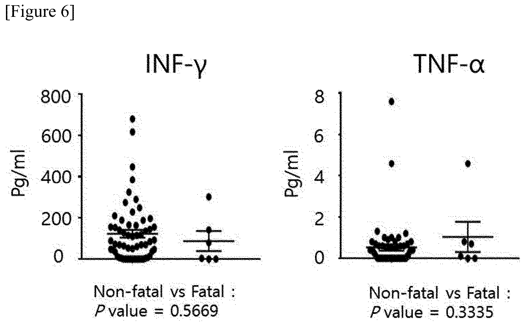

Also, as shown in Table 4 below and FIGS. 4 to 6, among the SFTS patients, the serum IL-6 and IL-10 concentrations were significantly higher in the fatal disease patients than the non-fatal disease patients, and the TGF-β concentrations were significantly lower in the fatal disease patients during the early clinical course of hospitalization (FIG. 4). However, similar to the results of COVID-19 patients and SFTS patients in previous studies, there was no statistically significant difference in the plasma IL-2, IL-4, IL-17A, IFN-γ, and TNF-α levels between the non-critical and fatal disease patients (FIGS. 5 and 6).

**[Table 4]**

| Characteristics | Non-fatal Disease Patients | Fatal Disease Patients | p-Value |
|---|---|---|---|
| IL-2 | 2.0 (0.0 - 26.7) | 0.6 (0.0 - 2.1) | 0.5498 |
| IL-4 | 0.7 (0.0 - 7.7) | 0.4 (0.0 - 1.0) | 0.6634 |
| IL-6 | 18.5 (0.0 - 325.7) | 3,151.2 (17.2 - 15,103.8) | < 0.0001 |
| IL-10 | 7.8 (0.3 - 72.3) | 42.9 (7.5 - 145.2) | < 0.0001 |
| IL-17A | 26.8 (0.0 - 142.5) | 16.0 (1.5 - 43.3) | 0.3722 |
| IFN-γ | 122.2 (0.0 - 679.6) | 87.3 (0.0 - 300.2) | 0.5669 |
| TNF-α | 0.5 (0.0 - 7.6) | 1.0 (0.0 - 4.6) | 0.3335 |
| TGF-β | 502.0 (25.2 - 1676.9) | 208.4 (28.7 - 422.8) | 0.0390 |

| | | | |
|---|---|---|---|
| Units: pg/mL; Unless otherwise stated, values are listed as median and range. | | | |

In addition, the dynamics of IL-6, IL-10, and TGF-β levels between non-fatal severe COVID-19 patients and non-fatal SFTS patients were compared. The results are shown in Tables 5 and 6 below.

**[Table 5]**

| Patient Group | Severity | Outcome | Date | IL-10 | IL-6 | TGF-β |
|---|---|---|---|---|---|---|
| P01-20 | Severe | Recovered | 2020.01.01 | 5.41579287 | 161.3035991 | 284 |
| | | | 2020.01.08 | 3.15431368 | 52.22262498 | 484 |
| | | | 2020.01.11 | 1.12915562 | 11.51367782 | 744 |
| P02-20 | Severe | Recovered | 2020.01.04 | 1.12915562 | 8.53021991 | 1244 |
| P03-20 | Severe | Recovered | 2020.02.19 | 4.00653923 | 36.85728705 | 784 |
| | | | 2020.02.27 | 2.15049603 | 17.5358723 | 984 |
| P04-20 | Severe | Recovered | 2020.05.28 | 7.92994231 | 62.24983932 | 184 |
| | | | 2020.06.03 | 2.86874188 | 82.67071059 | 464 |
| P05-20 | Severe | Recovered | 2020.03.27 | 2.58225586 | 6.654551571 | 884 |
| | | | 2020.03.31 | 1.4235996 | 2.602928555 | 764 |
| | | | 2020.04.05 | 0.98079668 | 5.251305728 | 784 |
| P06-20 | Severe | Recovered | 2020.04.14 | 0.52882958 | 3.53823216 | 304 |
| | | | 2020.04.24 | 0.52882958 | 4.472566498 | 324 |
| P01-21 | Severe | Recovered | 2021.01.01 | 4.28936455 | 211.0109794 | 384 |
| | | | 2020.01.11 | 1.71568504 | 17.69535959 | 544 |
| P02-21 | Severe | Recovered | 2021.05.22 | 5.13483866 | 99.96973305 | 144 |
| | | | 2021.05.31 | 2.43862766 | 5.562935417 | 864 |
| P03-21 | Severe | Recovered | 2021.06.14 | 3.43908062 | 9.000127299 | 44 |
| | | | 2021.06.30 | 1.56989853 | 7.122910008 | 824 |

**[Table 6]**

| Patient Group | Severity | Outcome | Date | IL-10 | IL-6 | TGF-β |
|---|---|---|---|---|---|---|
| P01-20 | Severe | Recovered | 2020.05.18 | 13.25898209 | 43.30547197 | 161.0382514 |
| | | | 2020.05.21 | 12.42552306 | 42.64772002 | 132.0765027 |
| | | | 2020.05.23 | 4.656795271 | 23.14924061 | 97.10382514 |
| | | | 2020.05.25 | 8.833360699 | 4.355882231 | 448.1967213 |
| P02-20 | Moderate | Recovered | 2020.05.27 | 7.532524704 | 7.984084078 | 258.852459 |
| | | | 2020.05.29 | 32.15189461 | 15.97059212 | 84.53551913 |
| | | | 2020.05.31 | 76.28075806 | 22.07534843 | 136.4480874 |
| | | | 2020.06.02 | 21.75896142 | 12.59148619 | 109.9453552 |
| | | | 2020.06.04 | 2.334776812 | 15.52607045 | 104.4808743 |
| | | | 2020.06.06 | 1.942924093 | 3.16201746 | 224.9726776 |
| | | | 2020.06.07 | 3.673326827 | 9.687814609 | 390.273224 |
| | | | 2020.06.08 | 0 | 1.786782344 | 383.989071 |
| P01-21 | Mild | Recovered | 2021.04.28 | 4.742734091 | 5.410369209 | 533.4426 |
| | | | 2021.04.30 | 1.424165626 | 104.9918703 | 255.5738 |
| | | | 2021.05.04 | 1.007576769 | 48.22225576 | 826.3388 |

As shown in Tables 5 and 6 above, it can be seen that IL-10 increased before IL-6, while TGF-β decreased after IL-6.

Based on these results, it was confirmed that the serum IL-6, IL-10, and TGF-β levels were significantly correlated in the SARS-CoV-2 and SFTSV patients.

### Example 3: Role of IL-10 and IL-6 cytokines in infected cells

### 3-1. Preparation of macrophages and infectious agents

A human monocyte cell line THP-1 (ATCC TIB-202) was used to model macrophages. THP-1 cells were cultured in an RPMI-1640 medium (Gibco) supplemented with 10% fetal bovine serum (FBS) (Gibco), penicillin-streptomycin, 1 mM sodium pyruvate (Thermo Fisher Scientific), 0.1 mM non-essential amino acids (Thermo Fisher Scientific), and 55 µM 2-mercaptoethanol (Thermo Fisher Scientific), and maintained in a humidified 5% CO₂ 37°C incubator. The THP-1 cells were differentiated into a macrophage phenotype at a density of 2 to 4 × 10⁵ cells/mL in a Hydro Cell 6-multiwell plate (Cell Seed Inc.) treated with 100 ng/mL phorbol myristate acetate (PMA) (Sigma-Aldrich) for 24 hours, washed, and then suspended in a medium without PMA.

In this experiment, the changes in the concentrations of IL-10, IL-6, and TGF-β after LPS treatment, SARS-CoV-2 treatment, and SFTSV treatment were confirmed.

Lipopolysaccharide (LPS) is a lipopolysaccharide component present in the cell wall of Gram-negative bacteria, and may induce a cytokine storm. To induce a cytokine storm environment, macrophages were treated with LPS (Sigma-Aldrich, St Louis, USA) at a concentration of 2 µg/mL.

SARS-CoV-2/BA.1.1 was isolated from a nasopharyngeal swab collected from a COVID-19 patient. The virus was propagated and titrated in Vero E6 cells (ATCC CRL-1586) cultured in a Dulbecco's modified Eagle's medium (DMEM) (Gibco) supplemented with 2% FBS and 1% penicillin-streptomycin (Gibco).

SFTSV (GenBank Accession No. MN329148-MN329150) was isolated from a domestic SFTS patient, and was propagated and titrated in Vero E6 cells (ATCC CRL-1586) cultured in a Dulbecco's modified Eagle's medium (DMEM) (Gibco) supplemented with 10% FBS.

### 3-2. Treatment of LPS-induced THP-1-derived macrophages with antibody

THP-1 cells were divided into three different treatment groups: a control treated with LPS (10 µg/mL) (Sigma-Aldrich); a group treated with LPS (10 µg/mL) (Sigma-Aldrich) and an IL-6R polyclonal antibody (10 µg/mL) (Invitrogen); and a group treated with LPS (10 µg/mL, Sigma-Aldrich, St Louis, USA) and an IL-10RA polyclonal antibody (an antibody targeting a portion (aa 22 to 235) of the amino acid sequence of SEQ ID NO: 3) (10 µg/mL) (Invitrogen).

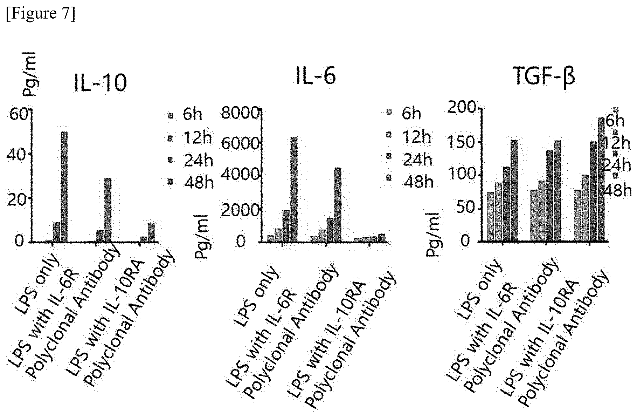

The three differently treated groups were stimulated for 6, 12, 24 and 48 hours, and the levels of IL-6 and IL-10 in the collected supernatant were measured using a human Th1/Th2/Th17 CBA kit (BD Biosciences) according to a slightly modified method from the manufacturer's instructions. Clinical specimens were obtained using a FACS Canto II flow cytometer and analyzed by FCAP Array software version 3.0 (BD Biosciences). The levels of TGF-β in the collected supernatant were measured using a human TGF-β ELISA kit (Thermo Fisher Scientific) according to the manufacturer's protocol. The results are shown in Table 7 below and FIG. 7.

**[Table 7]**

| Samples | Time (Hours) | IL-10 | IL-6 | TGF-β |
|---|---|---|---|---|
| LPS only | 6 | 0.66 | 356.75 | 73.41 |
| LPS only | 12 | 1.81 | 728.94 | 88.15 |
| LPS only | 24 | 12.22 | 1915.31 | 112.38 |
| LPS only | 48 | 57.98 | 8782.72 | 152.625 |
| LPS with IL-6R Polyclonal Antibody | 6 | 0 | 317.64 | 77.5 |
| LPS with IL-6R Polyclonal Antibody | 12 | 0 | 668.55 | 91.17 |
| LPS with IL-6R Polyclonal Antibody | 24 | 8.49 | 1353.59 | 136.565 |
| LPS with IL-6R Polyclonal Antibody | 48 | 32.09 | 6076.44 | 151.485 |
| LPS with IL-10RA Polyclonal Antibody | 6 | 0 | 184.44 | 77.48 |
| LPS with IL-10RA Polyclonal Antibody | 12 | 0.66 | 238.06 | 100.08 |
| LPS with IL-10RA Polyclonal Antibody | 24 | 4.13 | 284.37 | 150.05 |
| LPS with IL-10RA Polyclonal Antibody | 48 | 9.84 | 407.72 | 186.21 |

| | | | | |
|---|---|---|---|---|
| Units: pg/mL. | | | | |

As shown in Table 7 and FIG. 7, the correlation between IL-6, IL-10 and TGF-β in the LPS-induced THP-1-derived macrophages treated with IL-6R and IL-10RA polyclonal antibodies was as follows: when both groups were treated with an IL-10RA polyclonal antibody, the IL-6 level decreased and the TGF-β level increased. However, there was no significant difference between IL-6, IL-10 and TGF-β in the LPS-induced THP-1-derived macrophages treated with an IL-6R polyclonal antibody.

### 3-3. Investigation of role of IL-10 receptor in SARS-CoV-2-infected THP-1-derived macrophages

To investigate the role of IL-10 in SARS-CoV-2-infected THP-1 cells, THP-1 cells were infected with SARS-CoV-2 at a multiplicity of infection (MOI) of 0.01. Then, these SARS-CoV-2-infected THP-1 cells were divided into two different treatment groups: SARS-CoV-2-infected THP-1 cells as the control and SARS-CoV-2-infected THP-1 cells treated with an IL-10RA polyclonal antibody (10 µg/mL) (Invitrogen). The two different treatment groups were cultured for 6, 12, 24, and 48 hours, and the levels of IL-6 and IL-10 were measured using a human Th1/Th2/Th17 CBA kit (BD Biosciences) according to a slightly modified method from the manufacturer's instructions. Samples were obtained using a FACS Canto II flow cytometer, and analyzed by FCAP Array software version 3.0 (BD Biosciences), and TGF-β was measured in the collected supernatant using a human TGF-β ELISA kit (Thermo Fisher Scientific) according to the manufacturer's protocol.

**[Table 8]**

| Samples | Time (Hours) | IL-10 | IL-6 | TGF-β |
|---|---|---|---|---|
| SARS-CoV-2 only | 6 | 0.168 | 244.33 | 3.809 |
| SARS-CoV-2 only | 12 | 0 | 356.605 | 10.312 |
| SARS-CoV-2 only | 24 | 0.269 | 1401.53 | 31.637 |
| SARS-CoV-2 only | 48 | 1.824 | 4969.135 | 135.971 |
| SARS-CoV-2 with IL-10RA Polyclonal Antibody | 6 | 0 | 163.901 | 4.224 |
| SARS-CoV-2 with IL-10RA Polyclonal Antibody | 12 | 0 | 298.22 | 10.503 |
| SARS-CoV-2 with IL-10RA Polyclonal Antibody | 24 | 0.269 | 910.477 | 33.003 |
| SARS-CoV-2 with IL-10RA Polyclonal Antibody | 48 | 1.194 | 3217.858 | 144.328 |

| | | | | |
|---|---|---|---|---|
| Units: pg/mL. | | | | |

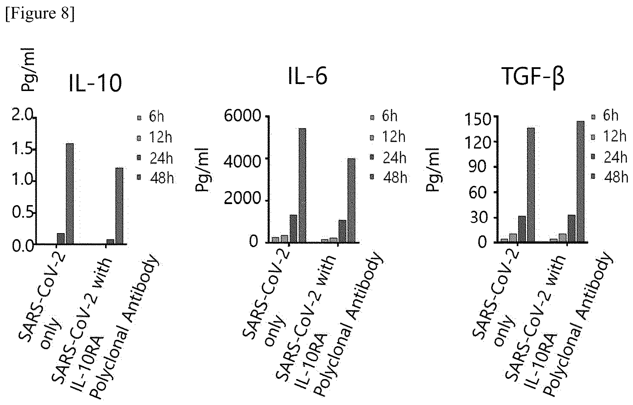

As shown in Table 8 and FIG. 8, when the THP-1-derived macrophages infected with SARS-CoV-2 were treated with an IL-10RA polyclonal antibody, the IL-6 levels decreased and the TGF-β levels increased, similar to the LPS-induced THP-1-derived macrophages.

### 3-4. Investigation of role of IL-10 receptor in SFTSV-infected THP-1-derived macrophages

To investigate the role of the IL-10 receptor in SFTSV-infected THP-1 cells, the THP-1 cells were infected with SFTSV at a multiplicity of infection (MOI) of 10. Thereafter, the SFTSV-infected THP-1 cells were divided into two different treatment groups; SFTSV-infected THP-1 cells as the control, and SFTSV-infected THP-1 cells treated with an IL-10RA polyclonal antibody (10 µg/ml) (Invitrogen). The two different treatment groups were cultured for 6, 12, 24, and 48 hours, and IL-6 and IL-10 levels were measured using a human Th1/Th2/Th17 CBA kit (BD Biosciences) according to a slightly modified method from the manufacturer's instructions.

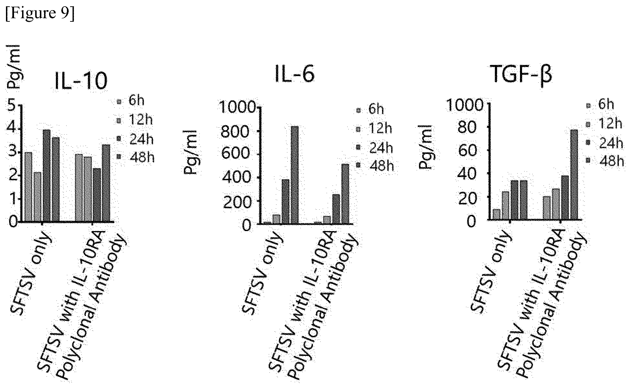

Samples were obtained using a FACS Canto II flow cytometer and analyzed by FCAP Array software version 3.0 (BD Biosciences). TGF-β was measured using a human TGF-β-1 ELISA kit (Thermo Fisher Scientific) according to the manufacturer's protocol. The results are shown in Table 9 below and FIG. 9.

**[Table 9]**

| Samples | Time (Hours) | IL-10 | IL-6 | TGF-β |
|---|---|---|---|---|
| SFTSV only | 6 | 3 | 16.28 | 9.125 |
| SFTSV only | 12 | 2.14 | 79.42 | 24.125 |
| SFTSV only | 24 | 3.96 | 380.08 | 33.5 |
| SFTSV only | 48 | 3.64 | 837.58 | 33.5 |
| SFTSV with IL-10RA Polyclonal Antibody | 6 | 2.9 | 18.26 | 20.375 |
| SFTSV with IL-10RA Polyclonal Antibody | 12 | 2.78 | 63.9 | 26.625 |
| SFTSV with IL-10RA Polyclonal Antibody | 24 | 2.3 | 255.36 | 37.875 |
| SFTSV with IL-10RA Polyclonal Antibody | 48 | 3.32 | 512.58 | 77.25 |

| | | | | |
|---|---|---|---|---|
| Units: pg/mL. | | | | |

As shown in Table 9 and FIG. 9, when SFTSV-infected THP-1-derived macrophages were treated with an IL-10RA polyclonal antibody, the IL-6 levels decreased and the TGF-β levels increased, similar to the LPS-induced THP-1-derived macrophages.

### Example 4: Comparison of effects of polyclonal antibodies

The same experiment was repeated with an IL-10RA polyclonal antibody (an antibody targeting amino acids 22 to 230 of the amino acid sequence of SEQ ID NO: 4) (designated as antibody 1) and an IL-10RA polyclonal antibody (an antibody targeting amino acids 22 to 235 of the amino acid sequence of SEQ ID NO: 3) (designated as antibody 2) (LSBIO) used in Example 3-2 to compare the effects.

THP-1 cells were divided into three different treatment groups: a control treated with LPS (10 µg/mL) (Sigma-Aldrich, St Louis, USA); a group treated with LPS and an IL-10RA polyclonal antibody (10 µg/mL) (Invitrogen); and a group treated with LPS and an IL-10RA polyclonal antibody (10 µg/mL) (Invitrogen).

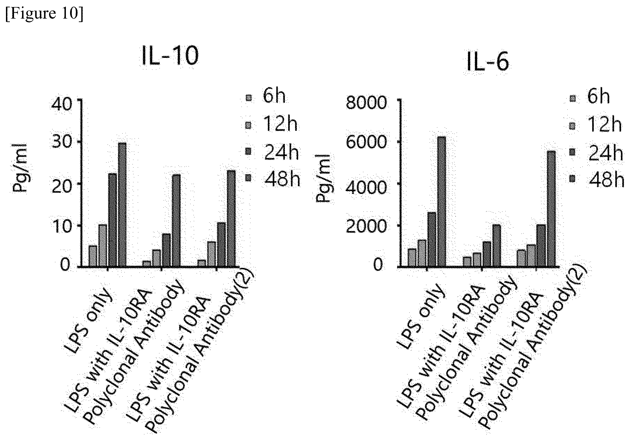

The three differently treated groups were stimulated for 6, 12, 24 and 48 hours, and the levels of IL-6 and IL-10 in the collected supernatant were measured using a human Th1/Th2/Th17 CBA kit (BD Biosciences) according to a slightly modified method from the manufacturer's instructions. Clinical specimens were obtained using a FACS Canto II flow cytometer and analyzed by FCAP Array software version 3.0 (BD Biosciences). The results are shown in Table 10 below and FIG. 10.

**[Table 10]**

| Samples | Time (Hours) | Antibody 1 | | Antibody 2 | |
|---|---|---|---|---|---|
| | | IL-10 | IL-6 | IL-10 | IL-6 |
| Control | 6 | 0.66 | 356.75 | 2.71 | 665.09 |
| Control | 12 | 1.81 | 728.94 | 6.98 | 1008.03 |
| Control | 24 | 12.22 | 1915.31 | 22.67 | 2064.24 |
| Control | 48 | 57.98 | 8782.72 | 25.95 | 4452.08 |
| IL-10R | 6 | 0.00 | 184.44 | 1.25 | 473.05 |
| IL-10R | 12 | 0.66 | 238.06 | 4.24 | 614.47 |
| IL-10R | 24 | 4.13 | 284.37 | 9.02 | 895.63 |
| IL-10R | 48 | 9.84 | 407.72 | 23.10 | 1525.16 |

The decreasing trend according to the type of polyclonal antibody appeared to be similar, and the antibody used as antibody 2 showed a similar effect to that of antibody 1. Therefore, the IL-10RA amino acid sequence (SEQ ID NO: 3) targeted by antibody 2 was used as the final target sequence to select a monoclonal antibody.

### Example 5: Selection of target site of IL-10RA polyclonal antibody

With reference to the amino acid sequence NP 001549.2 (Homo sapiens, interleukin-10 receptor subunit alpha precursor; SEQ ID NO: 1), the amino acid 22 to 230 sequence (SEQ ID NO: 4) that was the target of the antibody in Example 3 was selected. To this sequence, a sequence of 5 amino acids was added to select the amino acid 22 to 235 sequence (SEQ ID NO: 3) to manufacture a polyclonal antibody. Then, a monoclonal antibody was selected through efficacy comparison.

**[Table 11]**

| SEQ ID NO: | Name | Amino Acid Sequence (N-terminus --> C- terminus) | bp |
|---|---|---|---|
| 1 | Interleukin-10 receptor subunit alpha precursor | | 1-578 |
| | | | |
| 2 | IL-10RA partial amino acid sequence | mlpclvvlla allslrlgsd ahgtelpspp svwfeaeffh hilhwtpipn qsestcyeva llrygieswn sisncsqtls ydltavtldl yhsngyrarv ravdgsrhsn wtvtntrfsv devtltvgsv nleihngfil gkiqlprpkm apandtyegi fshfreyeia irkvpgnftf thkkvkhenf slltsgevge fcvqvkpsva srsnkgmwsk eecisltrqy ftvtn | 1-235 |
| 3 | IL-10RA amino acid sequence targeted by antibody 2 | hgtelpspp svwfeaeffh hilhwtpipn qsestcyeva llrygieswn sisncsqtls ydltavtldl yhsngyrarv ravdgsrhsn wtvtntrfsv devtltvgsv nleihngfil gkiqlprpkm apandtyegi fshfreyeia irkvpgnftf thkkvkhenf slltsgevge fcvqvkpsva srsnkgmwsk eecisltrqy ftvtn | 22-235 |
| 4 | IL-10RA amino acid sequence targeted by antibody 1 | hgtelpspp svwfeaeffh hilhwtpipn qsestcyeva llrygieswn sisncsqtls ydltavtldl yhsngyrarv ravdgsrhsn wtvtntrfsv devtltvgsv nleihngfil gkiqlprpkm apandtyegi fshfreyeia irkvpgnftf thkkvkhenf slltsgevge fcvqvkpsva srsnkgmwsk eecisltrqy | 22-230 |

## Claims

1. A pharmaceutical composition for preventing or treating an inflammatory disease, comprising, as an active ingredient, an antibody or an antigen-binding fragment thereof that specifically binds to an interleukin-10 (IL-10) receptor protein.

2. The pharmaceutical composition of claim 1, wherein the antibody or antigen binding fragment thereof specifically binds to an amino acid sequence of SEQ ID NO: 2 in the IL-10 receptor protein.

3. The pharmaceutical composition of claim 1, wherein the antibody or antigen binding fragment thereof specifically binds to an amino acid sequence of SEQ ID NO: 3 in the IL-10 receptor protein.

4. The pharmaceutical composition of claim 1, wherein the antibody or antigen binding fragment thereof specifically binds to an amino acid sequence of SEQ ID NO: 4 in the IL-10 receptor protein.

5. The pharmaceutical composition of claim 1, wherein the antibody or antigen binding fragment thereof is a polyclonal antibody or monoclonal antibody.

6. The pharmaceutical composition of claim 1, wherein the antigen binding fragment is scFv, (scFv)2, Fv, Fab, Fab', Fv, F(ab')2, a diabody, a triabody, a tetrabody, Bis-scFv, a nanobody, or a combination thereof.

7. The pharmaceutical composition of any one of claims 1 to 6, wherein the inflammatory disease is a disease caused by one or more selected from the group consisting of severe acute respiratory syndrome coronavirus 2 (SARS-CoV-2), severe fever with thrombocytopenia syndrome (SFTS) virus, and highly pathogenic avian influenza A (H5N1).

8. A use of an antibody or antigen binding fragment thereof that specifically binds to an interleukin-10 (IL-10) receptor protein for the preparation of a drug for preventing or treating an inflammatory disease.

9. A method of preventing or treating an inflammatory disease, comprising:
administering a therapeutically effective amount of an antibody or an antigen binding fragment thereof, which specifically binds to an interleukin-10 (IL-10) receptor protein, to a subject.
